# EUROPÄISCHE PATENTSCHRIFT

(11) **EP 1 046 908 B1**
(45) Veröffentlichungstag und Bekanntmachung des Hinweises auf die Patenterteilung: **26.05.2010**
(21) Anmeldenummer: 00106816.2
(22) Anmeldetag: 30.03.2000
(51) Int. Cl.: G01N 33/03, G01N 27/22

(54) **Vorrichtung zum Messen des Zustands von Ölen und Fetten**
Device for measuring the condition of oils and fats
Dispositif de mesure de la condition d'huiles et de graisses

(30) Priorität: 22.04.1999 DE 19918213
(43) Veröffentlichungstag der Anmeldung: 25.10.2000
(73) Patentinhaber: ebro Electronic GmbH & Co. KG, 85055 Ingolstadt (DE)
(72) Erfinder: Klün, Wolfgang, 85049 Ingolstadt (DE); Geul, Willem, 7623 CS Borne (NL)
(74) Vertreter: Bergmeier, Werner

(56) Entgegenhaltungen:
- EP-A- 0 389 916
- EP-A- 0 481 272
- DE-A1- 19 706 486
- US-A- 4 864 850
- US-A- 5 592 098
- US-A- 5 818 731
- IRION E ET AL: "Oil-Quality and Oil-Level Detection with the TEMIC QLT-Sensor Leads to Variable Maintenance Intervals" SAE TECHNICAL PAPER SERIES, SOCIETY OF AUTOMOTIVE ENGINEERS, WARRENDALE, PA, US, Nr. 970847, 1997, Seiten 105-110, XP002968686 ISSN: 0148-7191
- WABY G T: "An aeration meter for lubricating oils" JOURNAL OF SCIENTIFIC INSTRUMENTS, Bd. 42, Nr. 6, Juni 1965 (1965-06), Seiten 425-427, XP002320138

## Beschreibung

Die vorliegende Erfindung betrifft eine Vorrichtung zum Messen des Zustandes von Ölen oder Fetten gemäß dem Oberbegriff des Patentanspruchs 1.

Öle und Fette sind sowohl für den menschlichen Verzehr als auch insbesondere für die Zubereitung von Nahrungsmitteln von großer Bedeutung. So werden in heißen Ölen oder Fetten viele Nahrungsmittel, beispielsweise Kartoffeln oder bereits panierte Lebensmittel, durch Einlegen in diese heißen Fette gegart und damit dem menschlichen Verzehr zugänglich gemacht. (Unter Fetten sollen dabei insbesondere die feste Form von Ölen verstanden werden.) Zum Zwecke des Garens von Lebensmitteln werden die Öle und Fette in einem Temperaturbereich von ca. 90° Celsius bis 180° Celsius und mehr eingesetzt. Insbesondere diese heißen Temperaturen führen zu einer während des Einsatzes des Öles immer weiter ansteigenden Zerstörung bzw. Veränderung des Fettes.

Diese Veränderung ist eine Verschlechterung und findet im wesentlichen durch die Oxidation des Öles oder Fettes statt. Dabei entstehen viele chemische Produkte, wie z.B. freie Fettsäuren oder Polymere, die nicht nur den Geschmack der zubereiteten Gerichte negativ beeinflussen, sondern vor allem auch krankmachende Stoffe enthalten, die es erfordern, solche Öle, also insbesondere Fritierfette, regelmäßig und rechtzeitig auszutauschen. Im wesentlichen findet dieser Austausch nach Kriterien statt, die nicht direkt im Zusammenhang mit der chemischen Veränderung, insbesondere der schädlichen chemischen Veränderung, des Fettes stehen. Der Austausch erfolgt z.B. nach Ablauf einer bestimmten Zeit oder nach anderen irrelevanten Kriterien. So kommt es in der Praxis sowohl vor, dass die Fette zu früh als auch zu spät ausgetauscht wurden. Ersteres verursacht unnötige Kosten, wobei ein zu später Austausch die oben angeführten Gefahren in sich birgt.

Aus der US Patentschrift 3,739,265 ist ein Testinstrument bekannt, das Fett mittels eines Sensors daraufhin überprüft, ob es für den Einsatz noch brauchbar ist. Dabei ist vorgesehen, dass das Öl auf seine elektrischen Eigenschaften, insbesondere seine Dielektrizitäts-Eigenschaften hin, untersucht wird. Das Testgerät besitzt dazu eine schüsselförmige Aufnahme, die an ihrem Grund einen Sensor angeordnet hat, der als Kapazitätssensor ausgebildet ist. Dazu wird eine bestimmte Menge Öl auf den Sensor aufgebracht und die mit diesem System gemessene Kapazität in einem elektrischen Schaltkreis verarbeitet, wodurch man einen Wert erhält, der eine Aussage über den Zerstörungsgrad des Fettes ergibt. Dazu wird ein Vergleich durchgeführt zwischen dem zu testenden Fett bzw. Öl und einer Standardflüssigkeit, die bei einem Messvorgang jeweils ebenfalls gemessen werden muss. Die Änderung der Kapazität des Sensors ist ein Maß für den Zerstörungsgrad des Öles.

Aus der US Patentschrift 5,824,889 ist ein kapazitiv arbeitender Ölsensor zur Messung der Verschlechterung und Verschmutzung des Öls bekannt. Dieser findet Anwendung bei der Überprüfung des Öls von Verbrennungsmotoren. Die elektrischen Eigenschaften des Motorenöles bilden einen Anhaltspunkt für den Abbau des Öles bzw. für seine noch ausreichende Qualität. Die Dielektrizitätskonstante einer bestimmten Ölmarke verändert sich dabei innerhalb ganzer bestimmter Grenzen, die durch Versuche ermittelt wurden, und ergibt damit ein Maß für die Verschlechterung des Öles.

Darüber hinaus kann Motorenöl auch durch andere Faktoren in seiner Qualität verschlechtert sein, z.B. durch das Vorhandensein von Verschmutzungen, z.B. durch Kühlflüssigkeiten oder Benzin. Dies kann wiederum ein Maß dafür sein, wie weit der Verschleiß des Motors fortgeschritten ist und welcher Art er ist. Da beispielsweise Kühlflüssigkeit eine wesentlich höhere Dielektrizitätskonstante hat, kann eine wesentlich höhere Veränderung der Dielektrizitätskonstanten einen Hinweis auf Motorverschleiß bieten.

Der in der US-PS 5,824,889 beschriebene Sensor zum Messen der Eigenschaften des Öles von Verbrennungsmotoren besitzt eine Sensorfläche, die im Öl beispielsweise der Ölwanne eines Fahrzeuges montiert ist, wobei am Sensor nur ein schmaler Spalt vorgesehen ist, der den Zutritt von Öl zum Sensor gestattet.

Der Nachteil der erstgenannten Vorrichtung besteht darin, dass zur Messung des Öles dieses in eine Aufnahmevorrichtung eines Ölsensors eingefüllt werden muss. Dazu muss das Öl beispielsweise aus einer Friteuse entnommen werden und in die Messaufnahme des Sensors eingeführt werden. Nachdem dessen elektrische Eigenschaften gemessen wurden, wird das Öl wieder entfernt und die Messung mit einem Referenzöl zum Vergleich der ersten Messung durchgeführt. Da das Öl dabei in einer sehr kleinen Menge vorliegt, kühlt es sehr stark ab, wodurch bei Fetten die Gefahr besteht, dass diese hart werden und die Messung dadurch verfälscht oder unmöglich wird.

Darüber hinaus ist die Handhabung umständlich und das für die Messung verwendete Öl muss anschließend entsorgt werden, so dass insgesamt der Vorgang zeitaufwendig und unangenehm ist. Für den Einsatz in der Praxis, d.h. ohne großen Aufwand, ist dieses Gerät nicht geeignet. Zum Einsatz direkt in der Küche, um das verwendete Fett direkt und ohne Vorbereitungsarbeiten zu überprüfen, ist das Gerät nicht geeignet.

Der Sensor der US-PS 5,824,889 ist nicht nur, bedingt durch seine Einsatzzwecke, sehr klobig und nicht geeignet, ihn an verschiedenen Stellen zum Einsatz zu bringen, sondern er hat darüber hinaus den Nachteil, dass die Sensorfläche eine Abdeckung besitzt, die bei Einsatz des Sensors zu anderen Gelegenheiten einen Zutritt des Öles zum Sensor erschwert. Darüber hinaus wäre eine Reinigung des Sensors praktisch nicht möglich, da zu aufwendig. Insbesondere ein Einsatz in Verbindung mit Lebensmittelfetten scheidet dadurch aus.

Aus dem Artikel "Oil-Quality Prediction and Oil-Level Detection with the TE-MIC QLT-Sensor Leads to Variable Maintenance Intervals" aus der Zeitschrift "SAE Technical Paper Series, Sensors and Actuators, 1997 (SP-1220), S. 105-110, ISSN 0148-7191 ist ein kapazitiv arbeitender Sensor bekannt, der an einer Ölwannenwand eines Kraftfahrzeugs angebracht wird und zum Teil in die Ölwanne ragt, um die Ölqualität on-line zu messen. Vom Messkopf führt ein Kabel zur Messelektronik. Eine mögliche weitere Informationsübertragungskette ist nicht offenbart. Auch diese Vorrichtung ist dementsprechend unflexibel zu handhaben und nur für ein eng begrenztes Einsatzgebiet tauglich. Gleiches gilt im wesentlichen auch für die in der WO 98/50790 offenbarte Vorrichtung, die zur Anbringung an einer Ölwanne ausgebildet ist.

Die US Patentschrift 5,818,731 offenbart eine Vorrichtung zur Qualitätsmessung von Ölen oder Fetten zum Braten bzw. Kochen von Lebensmitteln, die in-situ-Messungen der Kapazität und der optischen Transmission kombiniert. In einem Ausführungsbeispiel befinden sich die Sensoren in einem Gehäuse, welches in die Ölwanne einer Fritteuse eingehängt wird; sie sind mit Kabeln mit einem Mikroprozessor verbunden, der die Sensorsignale auswertet und anzeigt, wenn das Öl ersetzt werden sollte.

Aufgabe der vorliegenden Erfindung ist es, eine Messvorrichtung vorzuschlagen, die die Nachteile des Standes der Technik vermeidet und geeignet ist, durch leichte Handhabbarkeit nicht nur zum Einsatz unter Laborbedingungen geeignet zu sein, sondern durch einfache Handhabung und Bedienbarkeit sehr variabel einsetzbar zu sein. Insbesondere der Einsatz der erfindungsgemäßen Vorrichtung im Bereich von Messungen, beispielsweise von Fritierölen oder -fetten, ohne dass diese erst zur Messung aus ihrer Pfanne entnommen werden müssen, soll geschaffen werden.

Darüber hinaus soll mit der Erfindung eine flexibel einsetzbare Vorrichtung geschaffen werden, die auch bei der Bestimmung der Qualität von anderen Ölen zum Einsatz kommen kann. Ziel der Erfindung ist es, auch eine Messvorrichtung mit einen Ölsensor zu schaffen, die mobil einsetzbar ist, d.h. ohne dass der Sensor zur Messung fest installiert oder im Ölbehälter integriert sein muss.

Die vorliegende Aufgabe wird durch die Merkmale des Anspruchs 1 gelöst.

Durch die erfindungsgemäße Ausgestaltung der Messvorrichtung wird erreicht, dass ein leicht handhabbares Gerät geschaffen wird, das einfach zu handhaben ist und auch bei Umgang mit heißen Flüssigkeiten sicher zu bedienen ist. Darüber hinaus hat sie den Vorteil, dass sie zuverlässige Messergebnisse liefert, unabhängig von der Temperatur des zu messenden Messgutes, und ohne dass Vorbereitungsarbeiten erforderlich sind, um das Gerät einsetzen zu können. Durch die Ausbildung des Ansatzes als stab- oder rohrförmiges Element wird vorteilhaft erreicht, dass Messkopf und Gehäuse starr miteinander verbunden sind, so dass zum Bedienen der Messvorrichtung nur eine Hand erforderlich ist. Die Handhabung von flexiblen Kabeln entfällt. Eine sichere Handhabung der Messvorrichtung ist gewährleistet. Darüber hinaus ermöglicht der Ansatz, dass dem Bediener der Messvorrichtung vorteilhaft ein sicherer Abstand zwischen zu messendem Messgut und seiner Hand, die die Messvorrichtung am Gehäuse führt, gewährleistet ist. Die Verbindung zwischen Messkopf und Auswerteelektronik erfolgt vorteilhaft über das Innere des Ansatzes. Gemäß der Erfindung ist einer Ausgabeeinheit der Messvorrichtung weiterhin ein Display zugeordnet, über das einfach optisch erkennbar ist, welche Parameter an der Messvorrichtung eingestellt werden und welches das Messergebnis ist. Besonders günstig wird dieses als Zahlenwert oder graphisch, z.B. als Bargraph dargestellt, wobei die Ausgabeeinheit derart ausgebildet ist, dass die Ausgabeeinheit einstellbar ist zur wahlweisen Darstellung des Messergebnisses.

Durch eine vorzugsweise Ausgestaltung der Messvorrichtung mit einem Kompensator wird vorteilhaft erreicht, dass Veränderungen des Sensors selbst, die durch eine Temperaturveränderung entstehen, kompensiert werden. Dadurch ist es einfach und kostengünstig möglich, die Messvorrichtung bei verschiedenen Temperaturen einzusetzen, ohne dass aufwendige Verfahren erforderlich wären, die ein Einstellen der Vorrichtung auf verschiedene Temperaturen erfordert.

In besonders vorteilhafter Weise ist der Kompensator von gleicher Bauart wie der Sensor selbst. Dadurch ist es besonders einfach und sicher möglich, den Kompensator in die Messelektronik schaltungstechnisch zu integrieren, ohne dass weitere zusätzliche Maßnahmen erforderlich wären. Besonders vorteilhaft ist der Kompensator ebenso wie der Sensor ausgestaltet. Dies gewährleistet eine sichere Aussage darüber, wie der Zustand des Öles oder Fettes ist. Der Sensor besitzt dazu vorteilhaft zwei Elektroden, von denen wenigstens eine aus einem dünnen Metalldraht besteht. Unter Draht wird dabei auch eine gedruckte Schaltung verstanden. Dazu wird z.B. Gold in Form einer Schaltung aufgebracht und anschließend auf der Trägerplatte fixiert. Dies kann beispielsweise durch Einbrennen erfolgen. Dies ermöglicht vorteilhaft, den Sensor wiederholgenau herzustellen und auch einfach und sicher auf einem Träger anzuordnen.

Ein Golddraht hat sich als besonders vorteilhaft erwiesen. Ganz besonders günstig ist die Trägerplatte aus Keramik ausgebildet, da diese chemisch neutral ist und gute Eigenschaften bezüglich der Wärmedehnung besitzt. Darüber hinaus hat sie den besonderen Vorteil, da die Messvorrichtung bei Lebensmitteln, wie z.B. Frittierfetten, eingesetzt werden soll, dass insbesondere Keramik lebensmittelecht, d.h. also unbedenklich im Einsatz mit Lebensmitteln ist. Besonders vorteilhaft wird für die Messvorrichtung ein Werkstoff verwendet, der bis über 200° Celsius hitzebeständig ist, insbesondere bis zu 230°. Dadurch ist ein sicherer Einsatz der Messvorrichtung gewährleistet, ohne dass diese durch die Temperatur des Messgutes zerstört wird.

In vorteilhafter Weise besitzt der Messkopf eine Abdeckung für den Sensor, so dass dieser nicht durch mechanische Berührungen zerstört werden kann.

Vorteilhaft ist diese Abdeckung so ausgebildet, dass sie als Kante des Ansatzes ausgebildet ist. Besonders vorteilhaft und einfach wird dies dadurch erreicht, dass der Ansatz zylinderförmig ausgebildet ist und mit einer Schräge endet. Dadurch wird der Sensor vor Berührungen geschützt und ist gleichzeitig insbesondere für Reinigungsmaßnahmen einfach zugänglich.

Günstigerweise ist die Messvorrichtung mit einer Auswerteelektronik ausgestaltet, der Speichermittel zugeordnet sind zur Speicherung von beispielsweise Konfigurationsdaten, Kalibrierdaten der Messvorrichtung oder Korrekturdaten. Dadurch kann vorteilhaft die Messvorrichtung insbesondere z.B. mittels spezifischer Daten zu bestimmten Ölen besonders exakt arbeiten. Dabei werden Daten aus Versuchsreihen in die Speichermittel integriert, so dass spezifische Abweichungen bzw. Veränderungen der Dielektrizitätskonstanten verschiedener handelsüblicher Fette und Öle berücksichtigt werden können, so dass die Messvorrichtung besonders genau arbeiten kann.

In besonders günstiger Ausgestaltung der Erfindung besitzt die Auswerte- und Steuerelektronik einen Microcontroller, der gewährleistet, dass die Messvorrichtung sicher, einfach, schnell und flexibel arbeiten kann. Mit Hilfe eines Microcontrollers sind insbesondere Korrekturdaten einfach zu handhaben und besonders genau. Erfindungsgemäß besitzt die Messvorrichtung dazu auch eine Eingabeeinheit für die Eingabe von Daten und eine Ausgabeeinheit für die Ausgabe des Messergebnisses, so dass der Messvorrichtung zusätzliche Informationen von der Bedienperson eingegeben werden können und dies beispielsweise auch über die Ausgabeeinheit kontrolliert werden kann, womit eine Vorrichtung geschaffen wird, die flexibel auf verschiedene Einsatzgebiete eingestellt und justiert werden kann.

Für die Speicherung von Daten besitzt die Messvorrichtung vorteilhaft ein EEPROM, was eine schnelle und umfangreiche Speicherung von Daten ermöglicht. In besonders vorteilhafter Weiterbildung der Erfindung besitzt der Messkopf noch zusätzlich einen Temperatursensor, so dass vorteilhaft die Vorrichtung gleichzeitig auch zur Messung der Temperatur des Messgutes eingesetzt werden kann. Vorteilhaft kann der Temperatursensor bzw. die gemessene Temperatur auch dazu eingesetzt werden, falls dies erforderlich ist, Korrekturwerte zu erstellen, die dann das Messergebnis noch genauer machen können.

Bei einem Verfahren zum Messen des Zustandes des Messgutes mit einer Kompensation temperaturbedingter Veränderungen des Messwertes des Sensors, welches selbst nicht Teil der Erfindung ist, wird erreicht, dass diese Kompensation einfach und sicher realisiert werden kann. Dadurch, dass die Kompensation elektronisch direkt in der Messbrücke erfolgt, sind aufwendige zusätzliche Maßnahmen nicht erforderlich. Die Elektronik ist dadurch einfach so ausgestaltet, dass sie sich selbst korrigiert. Aufwendige Steuerungs- und Regelungsmaßnahmen innerhalb einer Auswerteelektronik entfallen. Dadurch, dass vorteilhaft das Kompensationsglied ebenfalls als Kondensator ausgebildet ist, wird die Kompensation sicher erreicht. In vorteilhafter Weiterbildung des Verfahrens wird während der Messung das Kompensationsglied gleichzeitig derselben Temperatur ausgesetzt, wie der Sensor selbst. Insbesondere wird dies vorteilhaft dadurch erreicht, dass das Kompensationsglied auf demselben Träger wie der Sensor selbst angeordnet wird, wodurch sicher gewährleistet ist, dass der Kompensator derselben Temperatur ausgesetzt ist.

Besonders vorteilhaft wird eine Kompensation erreicht, wenn das Kompensationsglied gleich dem Sensor ausgebildet wird. Dadurch werden z.B. Fertigungsunterschiede und unterschiedliche Reaktionen eines elektronischen Bauteils durch eine andere räumliche Ausgestaltung ausgeschlossen. Dies macht das Verfahren besonders einfach und sicher und eine Vorrichtung kann besonders einfach hergestellt werden.

Besonders vorteilhaft wird zur Steuerung der Messbrücke und zur Erfassung der Werte der Messbrücke ein Microcontroller eingesetzt. Dies ermöglicht eine feine Steuerung der Messbrücke und Erfassung der Messergebnisse.

Eine entsprechende Vorrichtung zur Durchführung des soeben beschriebenen Verfahrens gewährleistet dessen sichere Durchführung. Besonders vorteilhaft wird die Vorrichtung ausgestaltet, wenn der Kompensator auf einem Träger angeordnet ist, der beispielsweise so mit dem Messgut in Berührung kommt, dass sich die Temperatur des Messgutes auch sicher auf den Kompensator überträgt. Besonders einfach und vorteilhaft wird die Vorrichtung dadurch ausgestaltet, dass ein Träger zum Einsatz kommt, der auf der einen Seite den Sensor und auf der Rückseite den Kompensator trägt. Dadurch erfassen beide über den Träger exakt die Temperatur des Messgutes. Dies ermöglicht eine sehr genaue Messung.

Durch die baugleiche Ausgestaltung von Kondensator und Kompensator wird das Verfahren durch die Vorrichtung sicher durchgeführt und es sind keine weiteren Maßnahmen und Vorrichtungen erforderlich, um eine Kompensation zu erreichen. Besonders vorteilhaft ist die Vorrichtung mit einem Microcontroller ausgestaltet, der die Messbrücke einfach und sicher und vielseitig steuert. Besonders vorteilhaft enthält dieser bereits Analog- und Digitalwandler, so dass keine zusätzlichen Bauteile erforderlich sind. Der Microcontroller enthält direkt Anschlüsse, die in der Lage sind, den von der Messbrücke gelieferten analogen Messwert direkt in einen digitalen Wert umzuwandeln, der vom Microcontroller auch weiter verarbeitet werden kann. Umgekehrt ist es genauso vorteilhaft, wenn der Microcontroller gleichzeitig auch Anschlüsse besitzt, die zur Steuerung der Messbrücke die Steuersignale des Microcontrollers bereits in analoger Form liefern, so dass sie der Messbrücke zugestellt werden können.

In besonders vorteilhafter Weiterbildung der Erfindung bietet die Vorrichtung im Messkopf noch zusätzlich einen Temperatursensor, der sowohl eine zusätzliche Information zusammen mit der Ausgabeeinheit für die Bedienperson schafft. Darüber hinaus ist es möglich, dass die Temperaturwerte des Temperatursensors vom Microcontroller für Steuerungs- und Kompensationszwecke Verwendung findet.

Im folgenden wird die Erfindung mittels zeichnerischen Darstellungen erläutert. Es zeigen
- **Figur 1a**: eine erfindungsgemäße Messvorrichtung,
- **Figur 1b**: eine Seitenansicht von Figur 1 a,
- **Figur 2**: eine Messelektronik 7 für die Messvorrichtung von Figur 1,
- **Figur 3**: die Auswerte- und Steuerelektronik für die Messvorrichtung von Figur 1,
- **Figur 4**: die Eingabeeinheit der Vorrichtung nach Figur 1.

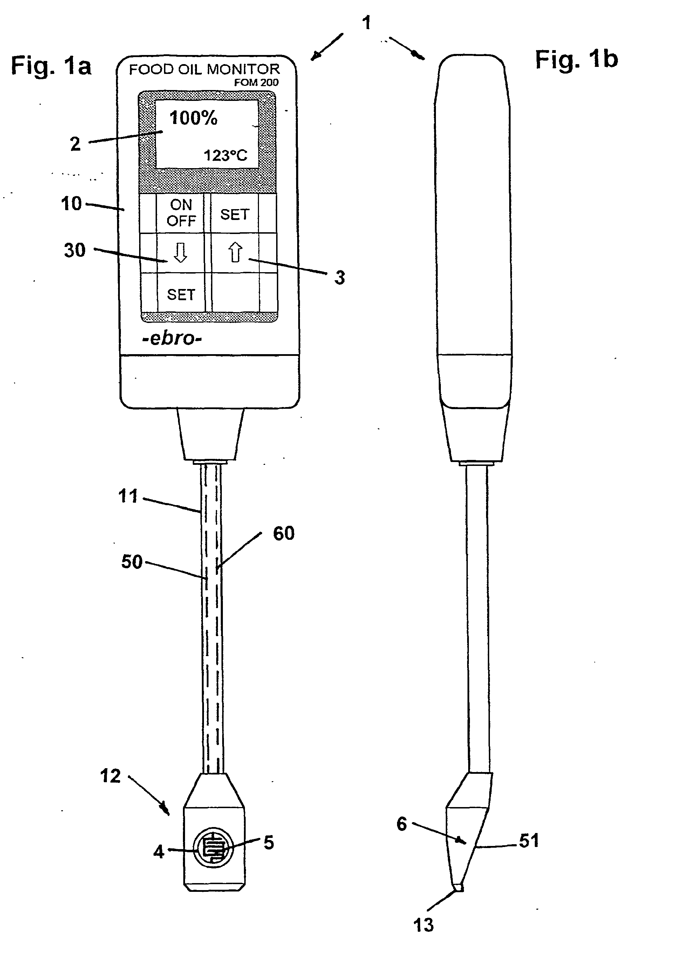

Figuren 1 a und 1 b zeigen eine erfindungsgemäß ausgestaltete Messvorrichtung 1 zum Messen des Zustandes von Ölen und Fetten. Die Messvorrichtung besteht aus einem Gehäuse 10, einem Ansatz 11 und einem Messkopf 12. Das Gehäuse 10 enthält, von außen sichtbar, eine Anzeige 2 für die Anzeige des Messwertes. Die Anzeige 2 ist in Form einer LCD-Anzeige ausgebildet und je nach Betrieb der Messvorrichtung 1 von verschiedenen Darstellungen, z.B. graphische Darstellung oder Darstellung mittels Zahlenwerten, umschaltbar. Das Umschalten auf verschiedene Arten der Anzeige erfolgt über die Eingabeeinheit 3, die in Form einer Folientastatur 30 ausgebildet ist. Die übrigen Teile des Gehäuses bilden gleichzeitig den Griff für das Erfassen der Messvorrichtung 1 durch eine Bedienperson.

An das Gehäuse 10 schließt sich der Ansatz 11 an, der eine Verbindung zwischen dem Messkopf 12 und dem Gehäuse 10 bildet. Der Ansatz 11 besteht Vorzugsweise aus einem dünnwandigen Rohr aus Edelstahl, z.B. aus V4A. Der Ansatz 11 hat die Aufgabe, eine Trennung zwischen Messkopf 12 und Gehäuse 10 mit der darin enthaltenen Steuerelektronik zu schaffen. Der Ansatz 11 ermöglicht es, die Messvorrichtung 1 als ein kompaktes Bauteil auszubilden, wo Messkopf 12 und Anzeigegerät mit Auswerteelektronik zwar räumlich voneinander getrennt sind, beide aber trotzdem mit einer Hand bedienbar sind.

Die im Stand der Technik üblichen Sensoren, die mittels eines Kabels mit ihrer Messelektronik verbunden sind, sind im Vergleich zur Messvorrichtung 1 wesentlich schwieriger handhabbar. Der Ansatz 11 hat gleichzeitig die Funktion, den Messkopf 12 so an der Messvorrichtung 1 zu halten, dass beim Einsatz der Messvorrichtung 1 ein derartiger Abstand zwischen Messkopf 12 und Gehäuse 10 vorhanden ist, dass die Auswerteelektronik innerhalb des Gehäuses 10 von der Temperatur des Messgutes ausreichend weit entfernt ist als auch die Hand der Bedienperson, die die Messvorrichtung 1 über das Gehäuse 10 während der Messung führt. Durch die Eigenschaft des Edelstahles ein schlechter Wärmeleiter zu sein, wird bei der Messvorrichtung 1 auch in Verbindung mit der Länge des Ansatzes 11 erreicht, dass auch bei einem längeren Verbleib des Messkopfes 12 in heißem Öl das Gehäuse 10 nicht durch die Temperatur belastet wird. Im Inneren des Ansatzes 11 verlaufen elektrische Leitungen, die den Messkopf 12 mit der Elektronik innerhalb des Gehäuses 10 verbinden.

Der Messkopf 12 besteht im wesentlichen aus einer Keramikplatte, die in das offene Ende des Ansatzes 11 eingesetzt ist. Die Keramikplatte 4 dichtet das untere Ende des Ansatzes 11 ab, da die Keramikplatte vollkommen dichtend in das Edelstahlrohr des Ansatzes 11 eingesetzt ist. Die Keramikplatte besitzt eine ovale Gestalt, da der rohrförmige Ansatz 11 mit einem Schnitt, der nicht senkrecht verläuft zur Achse des Ansatzes 11, abgeschnitten wurde. Der Schnitt und damit auch die Keramikplatte 4 verlaufen in etwa mit einem Winkel von 45° zur Längsachse des Ansatzes 11.

Auf der Keramikplatte 4 ist der Sensor 5 aufgebracht. Der Sensor 5 ist als kapazitiver Sensor ausgebildet, der zwei im wesentlichen gleiche Elektroden besitzt, die aus einem feinen in Schleifen gelegten Golddraht bestehen. Der Sensor 5 ist auf der dem Messgut zugewandten Seite 51 der Keramikplatte 4 aufgebracht. Er besteht aus Goldfäden, die durch ein thermisches Verfahren auf der Oberfläche aufgebracht sind. Auf der anderen Seite der Keramikplatte 4, in Figur 1a und 1b daher nicht sichtbar, ist in gleicher Weise ein zweiter Kondensator angebracht, der dieselbe Bauart wie der Sensor 5 besitzt und aus demselben Material besteht. Dieser auf der Rückseite der Keramikplatte 4 aufgebrachte Kondensator arbeitet als Kompensator 6. Sensor 5 und Kompensator 6 sind über zugehörige elektrische Leitungen 50 und 60 mit der elektronischen Schaltung der Messvorrichtung 1 verbunden. Der Kompensator 6 kommt mit dem Messgut nicht in Berührung.

Die Funktionsweise der Messvorrichtung 1 ist derart, dass über die Folientastatur 30 der Eingabeeinheit 3 von der Bedienperson das Gerät zunächst eingeschaltet wird. Nach dem Einschalten erfolgt ein Nullabgleich des Sensors zunächst gegen Luft, d.h. dass der Sensor noch nicht in das Messgut eingetaucht wird. Nach Freigabe durch die Steuereinheit, wobei dies über die Anzeige 2 angezeigt wird, kann der eigentliche Messvorgang beginnen. Dabei wird zunächst die Art des zu messenden Öles über die Eingabeeinheit 3 eingegeben und dann der Messkopf in die zu messende Flüssigkeit, also z.B. das heiße Öl, eingebracht. Nach ca. 10 Sekunden stabilisiert sich der Messwert in der Schaltung und wird vom Gerät, d.h. von einem Microcontroller, der sich in der Messvorrichtung 1 befindet, übernommen und das Messergebnis über das Display angezeigt, womit die Messung beendet ist. Während der Messung wird die Messvorrichtung 1 am Gehäuse 10 von der Bedienperson gehalten.

Der Sensor 5 kommt bei der Messung direkt in Kontakt mit dem heißen Fett und misst dadurch die Dielektrizitätskonstante des flüssigen Fettes bzw. Öles. Diesen besonders günstigen Vorteil der erfindungsgemäßen Messvorrichtung gewährleistet eine besonders einfache und jederzeit einzusetzende Vorrichtung. Es muss nicht abgewartet werden, bis das Messgut abgekühlt ist. Dies macht es auch einsetzbar für Fette, die bei höheren Temperaturen erst flüssig sind. Selbst während des laufenden Betriebs einer Fritüre ist die Messvorrichtung einsetzbar. Damit der Sensor 5 vor Beschädigungen geschützt ist, besitzt der Messkopf eine Abdeckung 13 für den Sensor 5. Im vorliegenden Ausführungsbeispiel ist die Abdeckung 13 dadurch gestaltet, dass der rohrförmige Ansatz 11 an seiner Spitze, die den Messkopf 12 trägt, schräg zur Achse des Ansatzes 11 ausgebildet ist.

Diese Art stellt auf der Keramikplatte 4 eine Fläche für den Sensor 5 zur Verfügung, die praktisch nicht mit einer Wandung eines Behälters in Kontakt kommt, da die Abdeckung 13 entweder eine Behälterwand oder den Behälterboden zuerst berührt, so dass der Sensor 5 damit nicht in Berührung kommen kann. Gleichzeitig bildet diese Form der Abdeckung 13 eine ausgezeichnete Möglichkeit, den Sensor 5 trotzdem frei zugänglich zu machen, z.B. zu Reinigungszwecken der Keramikplatte 4 durch den Benutzer der Messvorrichtung 1.

Der Sensor 5 in Form der Goldelektroden, die auf der Keramikplatte 4 fest aufgebracht sind, ist gegenüber Reinigungsmaßnahmen unempfindlich. Berührungen des Sensors 5 als solche sind also nicht generell schädlich, so dass die Abdeckung 13, wenn sie als Spitze, wie im vorliegenden Beispiel, ausgebildet ist, vollkommen ausreicht, um den Sensor 5 zu schützen. Wird ein empfindlicherer Sensor verwendet, werden an die Abdeckung 13 allerdings erhöhte Anforderungen gestellt, so dass dann beispielsweise noch Rippen oder sonstige Maßnahmen an der Keramikplatte 4 zu treffen sind, um einen ausreichenden Schutz für den Sensor 5 zu gewährleisten.

Durch die vorliegende Ausgestaltung des Sensors 5 und der Abdeckung 13 ist die Messvorrichtung 1 also unempfindlich und für den Einsatz in der Praxis bestens geeignet. Die Messvorrichtung 1 kann also praktisch wie ein Kochlöffel in einen Behälter gestellt werden, in dem die Messvorrichtung 1 mit ihrer Spitze, d.h. der Abdeckung 13, auf dem Boden des Behälters aufsteht. Der Ansatz 11, der, wie oben bereits beschrieben, aus einem Rohr aus Edelstahl besteht, trägt ebenfalls zur Unempfindlichkeit und zur Praxistauglichkeit der Messvorrichtung 1 bei. Der Ansatz 11 hat dazu und zur Abschirmung der Hitze des Messgutes eine Länge zwischen 15 cm und 40 cm, vorzugsweise eine Länge zwischen 25 cm und 35 cm. Der Durchmesser des rohrförmig ausgebildeten Ansatzes 11 hat dabei vorteilhaft einen Durchmesser zwischen 10 mm und 20 mm.

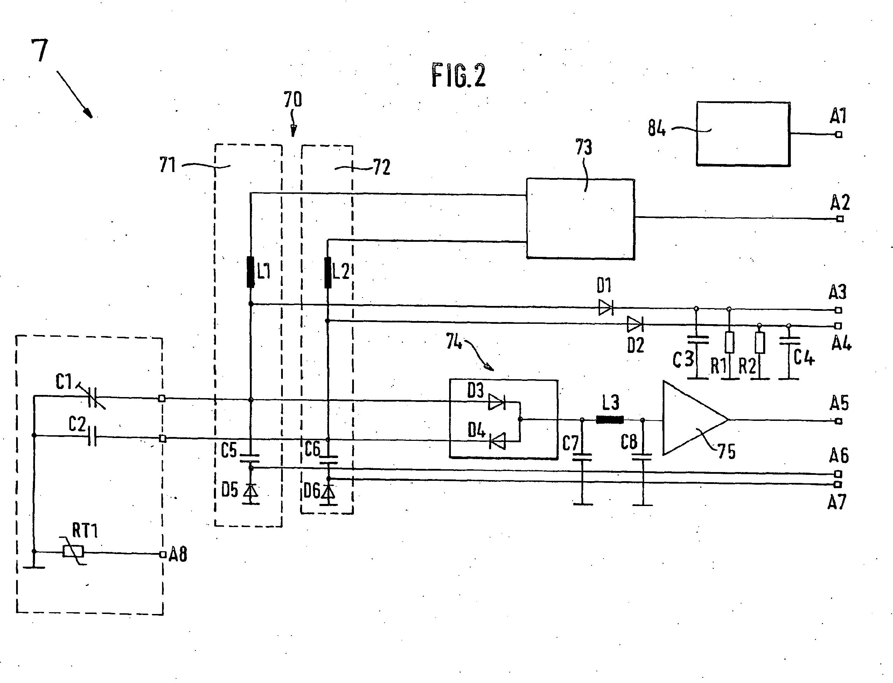

Figur 2 zeigt die wesentlichen Elemente der Messelektronik 7, mit deren Hilfe die Dielektrizitätskonstante des zu messenden Öles bzw. Fettes bestimmt wird. Die Messelektronik 7 besteht aus einem Ölresonanzkreis 71 mit dem dazugehörigen, als Kondensator ausgebildeten, Sensor C1. Dieser Ölresonanzkreis bildet einen Teil der Messbrücke 70 der Messelektronik 7. Den zweiten Teil der Messbrücke 70 bildet der Kompensationsresonanzkreis 72. Der Ölresonanzkreis wird aus dem mit dem Öl in Kontakt befindlichen Kondensator C1 (Sensor C1) gebildet, außerdem dem Kondensator C5, der Kapazitätsdiode D5 und der Induktivität L1.

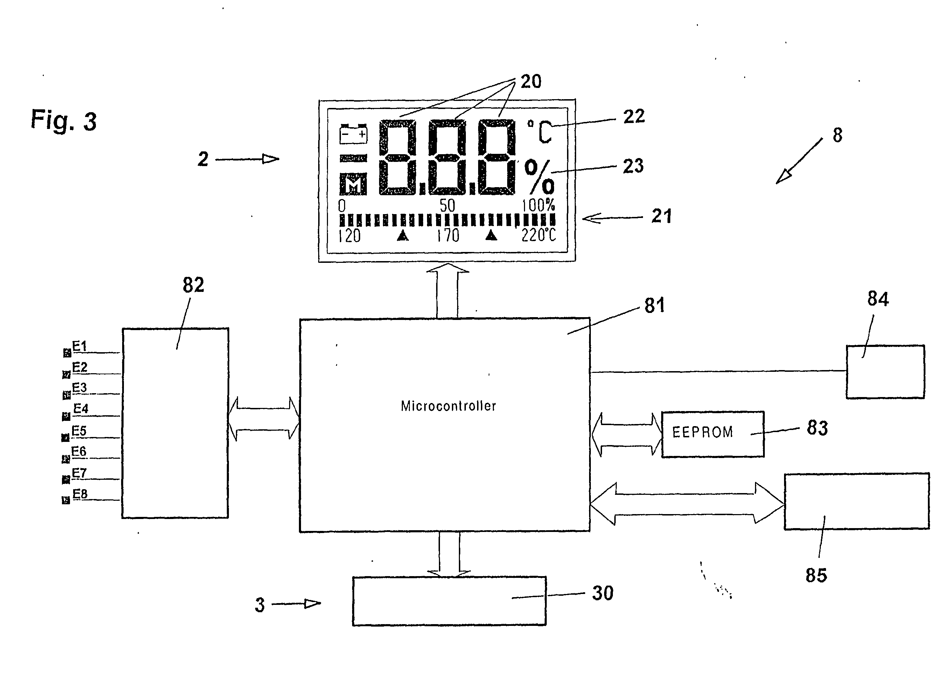

Der Kompensationsresonanzkreis besteht aus dem Kondensator C2 (Kompensator C2), dem Kondensator C6, der Kapazitätsdiode D6 und der Induktivität L2. Beide Resonanzkreise 71, 72 werden aus einem von einem Microcontroller (vergleiche Figur 3) mit einer Hochfrequenz von etwa 1 MHz bis 100 kHz gespeist. Es hat sich aber auch gezeigt, dass besonders günstig auch eine Frequenz im Bereich von ca. 50 kHz verwendet werden kann. Die hochfrequente Wechselspannung wird von einem programmierbaren Oszillator 73 in die Messbrücke 70 eingespeist. Um die Resonanzcharakteristik der beiden Brückenzweige 71, 72 steuern zu können, wird den beiden Kreisen eine einstellbare Gleichspannung auf die Kapazitätsdioden D5 und D6 gegeben. Diese ändern dadurch ihre Kapazität und damit die Resonanzfrequenz des dazugehörigen Brückenzweiges.

Um die Wirkung der Gleichspannung kontrollieren zu können, ist für beide Resonanzkreise 71, 72 je ein Amplitudengleichrichter in der Schaltung enthalten. Der Amplitudengleichrichter für den Ölresonanzkreis 71 besteht aus der Diode D1, der Kapazität C3 und dem Widerstand R1. Für den Kompensationsresonanzkreis 72 besteht der Amplitudengleichrichter aus der Diode D2, dem Kondensator C4 und dem Widerstand R2. Die Resonanzcharakteristik der Brückenzweige wird durch den Microcontroller gesteuert, der über die Anschlüsse A6 und A7 mit einer Gleichspannung auf die Kapazitätsdioden D5 und D6 eingreift. Die Resonanzcharakteristik der Messbrücke 70 erfasst der Microcontroller über die Anschlüsse A3 und A4. Die Steuerung des Oszillators 73 erfolgt über den Anschluss A2.

Die Dioden D3 und D4 bilden zusammen mit den Kapazitäten C7 und C8 und der Induktivität L3 einen Phasendiskriminator 74, der über einen darauf folgenden Verstärker 75 das eigentliche Signal für den gewünschten Messwert am Anschluss A5 zur Verfügung stellt. Besonders günstig ist es dabei, wenn der durch den Kondensator (C1) gemessene Kapazitätsunterschied von ca. 10⁻¹⁵ F zwischen dem unverbrauchten Öl und dem verbrauchten Öl oder Fett, einen Spannungsunterschied von 100 mV ergibt. Um dies zu erreichen, wird günstigerweise ein Sensor (C1) mit einer Sensorkapazität zwischen ca. 0,1 pF und 50 pF, vorzugsweise zwischen 1 pF und 5 pF verwendet.

Der Messkopf 12 enthält einen Öltemperatursensor RT1, der über die Messelektronik 7 elektrisch versorgt ist. Darüber hinaus greift er (RT1) in die Schaltung der Messbrücke 70 nicht ein. Über den Anschluss A8 wird vom Microcontroller (vergleiche Figur 3) über eine Sensorschnittstelle die Öltemperatur abgegriffen. Die Stromversorgung erfolgt über den Anschluss A1.

Alternativ können die Resonanzkreise sowie die Kapazitätsdioden bereits in einem integrierten Baustein enthalten sein.

Figur 3 zeigt eine schematische Darstellung der Auswerte- und Steuerelektronik 8 einer Vorrichtung zur Durchführung der Messung des Zustandes von einem Messgut, die insbesondere in einer Messvorrichtung, wie sie z.B. in Figur 1 beschrieben ist, eingesetzt wird. Die Auswerte- und Steuerelektronik 8 besteht im wesentlichen aus einem Microcontroller 81. Der Microcontroller 81 steht, über eine Sensorschnittstelle 82, mit der Messelektronik (vergleiche Figur 2) in Verbindung.

Die Anschlüsse A1 bis A8 besitzen entsprechende Eingänge E1 bis E8 an der Sensorschnittstelle 82 des Microcontrollers 81. Über eine Tastatur, insbesondere eine Folientastatur 30, steht der Microcontroller 81 mit der Außenwelt in Verbindung, so dass bestimmte Werte zum Steuern der Messvorrichtung 1 (vergleiche Figur 1) eingegeben werden können.

Zum Speichern von Daten, z.B. Messwerten oder Steuerdaten für die Korrektur des Messergebnisses oder sonstiger Steuerdaten, ist dem Microcontroller 81 ein EEPROM 83 zugeordnet. Darüber hinaus besitzt der Microcontroller 81 einen Anschluss für eine Stromversorgung, die insbesondere als Batterie 84 ausgebildet ist. Das Ausführungsbeispiel einer Messvorrichtung 1 von Figur 1 besitzt darüber hinaus einen Anschluss an einer Test- und Programmierschnittstelle, an der z.B. ein PC anschließbar ist. Die Test- und Programmierschnittstelle 85 ihrerseits ist mit dem Microcontroller 81 verbunden.

Ein ganz wesentliches Element der Auswerte- und Steuerelektronik 8 ist eine Anzeige 2, die vom Microcontroller 81 gesteuert wird. Über die Anzeige 2, die als LCD-Anzeige ausgebildet ist (vergleiche Figur 1), werden Messergebnisse und sonstige Daten vom Microcontroller 81 über das Display 2 der Bedienperson der Messvorrichtung zur Anzeige gebracht. Das LCD-Display der Anzeige 2 ist vorteilhaft übersichtlich und aussagekräftig so gestaltet, dass es eine numerische Anzeige 20 besitzt sowie einen Bereich, der eine Anzeige als Bargraph 21 ermöglicht. Über die numerische Anzeige kann wahlweise (vergleiche Beschreibung von Figur 1) die Temperatur des Öltemperatursensors RT1 (vergleiche Figur 2) numerisch angezeigt werden, ebenso wie der Zustand des Messgutes, d.h. eine Prozentangabe, zu wieviel Prozent das Messgut noch für die weitere Verwendung tauglich ist. So ist z.B. ein neues Öl mit dem Wert 100 gekennzeichnet, der die Aussage 100% repräsentiert.

Darüber hinaus kann die Information über die Temperatur des Öles und den Ölzustand auch über den Bereich, der einen Bargraphen 21 anzeigt, dargestellt werden. Der Bargraph 21 besteht aus einzelnen aneinandergereihten Rechtecken, die bei dem in Figur 3 gezeigten Testbild der Anzeige 2 alle schwarz dargestellt sind und somit einen Wert von 100% für den Zustand des Öles und eine Temperatur von ca. 240° Celsius des Öls repräsentieren.

Ist das Öl weiter verschlissen bzw. die Temperatur des Öles niedriger, beginnen die dunklen Rechtecke von rechts nach links sich aufzuhellen und dadurch zu verschwinden. Sind nur noch die Hälfte der Rechtecke von links nach rechts dunkel hinterlegt, repräsentieren sie einen Wert von 50% für das Öl, d.h. es ist nur noch zu 50% seines Maximalwertes in guter Kondition. Gleichzeitig kann der Bargraph damit die Temperatur von 170° Celsius anzeigen. Ob ein Prozentwert oder ein °C-Wert von der Anzeige 2 dargestellt wird, hängt davon ab, ob das °C-Zeichen 22 oder das %-Zeichen 23 dunkel hinterlegt ist.

Gleichzeitig zeigt das Display noch den Zustand der Stromversorgung der Messvorrichtung an. Diese Form der Anzeige, wie sie das Display der Anzeige 2 darstellt, ist besonders übersichtlich und passt sich den Vorstellungen der Bedienperson an. Die Umstellung zwischen einer %-Anzeige oder °C-Anzeige bzw. einer Anzeige über den Bargraphen 21 wird von der Bedienperson über die Folientastatur 30 ausgewählt.

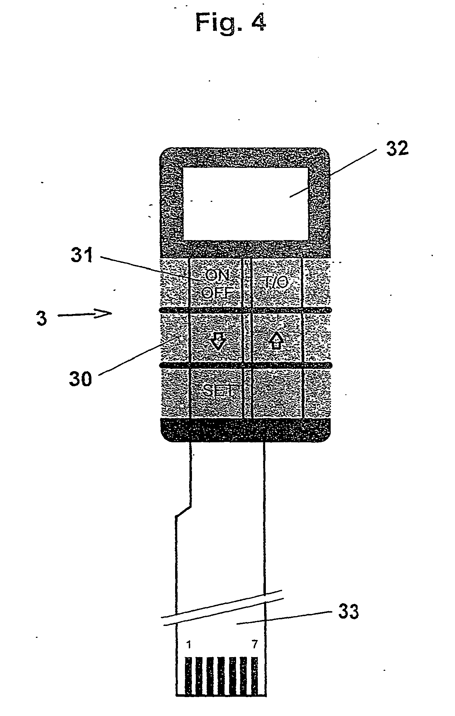

Figur 4 zeigt die Eingabeeinheit 3, die in Form einer Folientastatur 30 ausgebildet ist. Oberhalb der einzelnen Tasten 31 besitzt sie eine Aussparung 32, um die Anzeige 2 (vergleiche Figur 1) nicht zu überdecken. Die Folientastatur 30 besitzt einen Ein-/Ausschalter (On/Off), einen Wahlschalter (T/O) und einen Schalter zum Einstellen verschiedener Eingabe- oder Ausgabemodi (Set). Darüber hinaus besitzt sie noch zwei Eingabetasten (+ und -), mit denen im Auswahlmenü, das der Microcontroller zur Verfügung stellt, die einzelnen Modi durchgeblättert und ausgewählt werden können. Die Folientastatur 30 ist über einen Anschluss 33 an den Microcontroller 81 (vergleiche Figur 3) angeschlossen.

Die im einzelnen bei den verschiedenen Figuren und bei der Beschreibung der Vorrichtungen dargestellten Vorzüge der Erfindung können einzeln in einer Messvorrichtung verwirklicht werden oder vorteilhaft auch alle zusammen in einer Messvorrichtung, soweit dies rein praktisch möglich ist. Dies liegt im freien Ermessen des Fachmanns und den jeweiligen Anforderungen an eine Messvorrichtung. So kann es z.B. vorteilhaft sein, eine Messvorrichtung so auszugestalten, dass sie innerhalb der Auswerte- und Steuerelektronik nur einen Satz von Konfigurationsdaten besitzt, die nur für eine Sorte von Öl genaue Aussagen zulässt. Dadurch ist es möglich, das Gerät z.B. zusammen mit einer Sorte Öl oder Fett an den Endverbraucher auszuliefern, so dass dieser ohne Einstellmaßnahmen die von ihm verwendete Sorte Fett oder Öl auf seine Gebrauchseigenschaften untersuchen kann.

Darüber hinaus ist es auch möglich, falls dies erforderlich ist, die Messdaten des Öltemperatursensors RT1 im Microcontroller dazu zu benutzen, Konfigurationsdaten für die Bestimmung von Messwerten zur Verfügung zu stellen, wenn dies für eine exakte Zuordnung zur Temperatur des Messgutes erforderlich ist.

## Patentansprüche

1. Messvorrichtung (1) eingerichtet zum Messen des Zustandes eines Messgutes bestehend aus Ölen oder Fetten zur Verarbeitung von Lebensmitteln, mit einem Messkopf (12) zum Messen einer elektrischen Eigenschaft des Messgutes und mit einem Gehäuse (10), **dadurch gekennzeichnet, dass** der Messkopf (12) am freien Ende eines Ansatzes (11) des Gehäuses (10) angeordnet ist, dass im Gehäuse eine Auswerte- und Steuerelektronik (8) angeordnet ist, und dass der Ansatz (11) mit einer Länge zwischen 15 cm und 40 cm zur Verhinderung von thermischen Einflüssen, die durch die Messung auf den Messkopf (12) einwirken, auf die Auswerte- und Steuerelektronik (8) ausgebildet ist, wobei der Auswerte- und Steuerelektronik (8) eine Eingabeeinheit (3) für die Eingabe von Daten und eine Ausgabeeinheit (2) für die Ausgabe des Messergebnisses, zugeordnet ist, und wobei weiterhin der Ausgabeeinheit (2) ein Display zugeordnet ist zur Anzeige von über die Eingabeeinheit (3) eingegebenen Parametern und/oder zur Darstellung der Messergebnisse.

2. Messvorrichtung nach Anspruch 1, **dadurch gekennzeichnet, dass** der Ansatz (11) am Gehäuse (10) ein stab- oder rohrförmiges Element ist, das an dessen einer Seite mit dem Gehäuse (10) in Verbindung steht, und das an seiner anderen Seite den Messkopf (12) der Messvorrichtung trägt und temperaturbeständig ausgebildet ist.

3. Messvorrichtung nach Anspruch 1 oder 2, **dadurch gekennzeichnet, dass** der Ansatz (11) rohrförmig ausgebildet ist und in seinem Inneren Verbindungsmittel (50, 60) zum Verbinden des Messkopfes (12) mit der Auswerte- und Steuerelektronik (8) aufnimmt.

4. Messvorrichtung nach einem der Ansprüche 1 bis 3, **dadurch gekennzeichnet, dass** der Ansatz (11) eine Länge zwischen 25 cm und 35 cm aufweist.

5. Messvorrichtung nach einem der Ansprüche 1 bis 4, **dadurch gekennzeichnet, dass** der Ansatz (11) eine derartige Materialeigenschaft aufweist, dass die Temperatur am Messkopf (12) keine Auswirkung auf die Auswerte- und Steuerelektronik (8) hat.

6. Messvorrichtung nach einem der Ansprüche 1 bis 5, **dadurch gekennzeichnet, dass** der Messkopf (12) einen Sensor (5) zum Messen der elektrischen Eigenschaften des Messgutes trägt sowie einen Kompensator (6), welcher den Temperatureinfluss auf den Sensor (5) **dadurch** zu kompensieren vermag, dass er entsprechende Auswirkungen zu registrieren und an die Auswerte- und Steuerelektronik (8) weiterzuleiten vermag, wobei der Kompensator (6) eine Veränderung bei Änderung seiner Temperatur erfährt und diese Veränderung von der Auswerte- und Steuerelektronik (8) der Messvorrichtung erfasst wird.

7. Messvorrichtung nach Anspruch 6, **dadurch gekennzeichnet, dass** der Sensor (5) zur Messung der Dielektrizitätskonstanten des Messgutes ausgebildet ist.

8. Messvorrichtung nach Anspruch 7, **dadurch gekennzeichnet, dass** der Sensor (5) zum Messen der Dielektrizitätskonstanten zwei Elektroden besitzt, von denen wenigstens eine aus einem dünnen Metalldraht besteht, der isoliert auf einer Trägerplatte (4) angeordnet ist.

9. Messvorrichtung nach Anspruch 8, **dadurch gekennzeichnet, dass** der Metalldraht ein Golddraht ist.

10. Messvorrichtung nach Anspruch 8 oder 9, **dadurch gekennzeichnet, dass** die Trägerplatte (4) aus Keramik ausgebildet ist.

11. Messvorrichtung nach einem der Ansprüche 1 bis 10, **dadurch genkennzeichnet, dass** wenigstens der Ansatz (11), der Messkopf (12), die Trägerplatte (4) oder der Sensor (5) aus einem Werkstoff ausgebildet ist, der für die Verwendung in Verbindung mit Lebensmitteln zugelassen ist.

12. Messvorrichtung nach Anspruch 11, **dadurch gekennzeichnet, dass** der Werkstoff hitzebeständig bis 230°C ist.

13. Messvorrichtung nach einem der Ansprüche 6 bis 10, **dadurch gekennzeichnet, dass** der Messkopf (12) eine Abdeckung (13) für den Sensor (5) besitzt, die den Sensor (5) vor Berührung mit der Wand oder dem Boden eines Behälters für das Messgut schützt.

14. Messvorrichtung nach Anspruch 13, **dadurch gekennzeichnet, dass** die Abdeckung (13) als Kante ausgebildet ist, die den Sensor (5) überragt.

15. Messvorrichtung nach einem der Ansprüche 1 bis 14, **dadurch gekennzeichnet, dass** die Ausgabeeinheit (2) umschaltbar ausgestaltet ist zur wahlweisen Darstellung von Messergebnissen als Zahlenwert oder graphisch, beispielsweise als Bargraph.

16. Messvorrichtung nach einem der Ansprüche 1 bis 15, **dadurch gekennzeichnet, dass** der Auswerte- und Steuerelektronik (8) Speichermittel (83) zugeordnet sind zur Speicherung von beispielsweise Kalibrierdaten oder Korrekturdaten.

17. Messvorrichtung nach einem der Ansprüche 1 bis 16, **dadurch gekennzeichnet, dass** die Auswerte- und Steuerelektronik (8) einen Microcontroller (81) enthält.

18. Messvorrichtung nach Anspruch 17, **gekennzeichnet durch** eine Test- und Programmierschnittstelle (85), an der ein PC anschließbar ist, wobei die Test- und Programmierschnittstelle (85) ihrerseits mit dem Microcontroller (81) verbunden ist.

19. Messvorrichtung nach einem der Ansprüche 1 bis 18, **dadurch gekennzeichnet, dass** der Auswerte- und Steuerelektronik (8) zur Speicherung von Daten ein EEPROM (83) zugeordnet ist.

## Claims

1. A measuring device (1) for measuring the state of a material consisting of oils or fats for processing foods, said measuring device comprising a measuring head (12) for measuring an electric quality of the material to be measured and a housing (10), **characterized in that** the measuring head (12) is mounted on the free end of an attachment (11) of the housing (10), that an evaluation and control electronics (8) is arranged in the housing, and that the attachment (11) having a length between 15 cm and 40 cm is designed so that temperature influences on the measuring head (12) due to the measurement do not affect the evaluation and control electronics (8) ; wherein an input unit (3) for the input of data and an output unit (2) for the output of measuring result are assigned to the evaluation and control electronics (8) and wherein furthermore a display is assigned to the output unit (2) to show the parameter entered via the input unit (3) and/or to illustrate the measuring values.

2. The measuring device according to claim 1, **characterized in that** the attachment (11) on the housing (10) is a rod-shaped or tubular element connected on its one end to the housing (10) and carrying on the other end the measuring head (12) of the measuring device and being temperature-resistant.

3. The measuring device according to claim 1 or 2, **characterized in that** the attachment (11) is tubular and receives in its interior connecting means (50, 60) for connecting the measuring head (12) to the evaluation and control electronics (8).

4. The measuring device according to one of claims 1 to 3, **characterized in that** the attachment (11) has a length between 25 cm and 35 cm.

5. The measuring device according to one of claims 1 to 4, **characterized in that** the attachment (11) has a material quality such that the temperature on the measuring head (12) has no effect on the evaluation and control electronics (8).

6. The measuring device according to one of claims 1 to 5, **characterized in that** the measuring head (12) carries a sensor (5) for measuring said electric quality of the material to be measured and also carries a compensator (6) being capable of compensating the influence of temperature on the sensor (5) by registering appropriate effects and transmitting them to the evaluation and control electronics (8) wherein the compensator (6) is subject to a modification by a change of its temperature and said modification is registered by the evaluation and control electronics (8) of the measuring device.

7. The measuring device according to claim 6, **characterized in that** the sensor (5) is designed for measuring the dielectric constant of the material to be measured.

8. The measuring device according to claim 7, **characterized in that** the sensor (5) for measuring the dielectric constant comprises two electrodes at least one of which consists of a thin metal wire arranged in an insulated manner on a carrier plate (4).

9. The measuring device according to claim 8, **characterized in that** the metal wire is a gold wire.

10. The measuring device according to claim 8 or 9, **characterized in that** the carrier plate (4) consists of ceramic material.

11. The measuring device according to one of claims 1 to 10, **characterized in that** at least the attachment (11), the measuring head (12), the carrier plate (4) or the sensor (5) consist of a material licensed for being used in conjunction with foods.

12. The measuring device according to claim 11, **characterized in that** said material is heat resistant up to 230° C.

13. The measuring device according to one of claims 6 to 10, **characterized in that** the measuring head (12) comprises a cover (13) for the sensor (5) which protects the sensor (5) from making contact with a wall or a bottom of a container for the material to be measured.

14. The measuring device according to claim 13, **characterized in that** the cover (13) is formed as an edge extending over the sensor (5).

15. The measuring device according to one of claims 1 to 14, **characterized in that** the display (2) can be switched to selectively show measured results as a numeric value or graphically, e.g. as a bargraph.

16. The measuring device according to one of claims 1 to 15, **characterized in that** the evaluation and control electronics (8) is associated with storage means (83) for storing for instance calibration data or correction data.

17. The measuring device according to one of claims 1 to 16, **characterized in that** the evaluation and control electronics (8) comprise a microcontroller (81).

18. The measuring device according to claim 17, **characterized by** a test and programming interface (85) to which a PC can be connected, the test and programming interface (85) on its part being connected to the microcontroller (81).

19. The measuring device according to one of claims 1 to 18, **characterized in that** an EEPROM (83) is associated with the evaluation and control electronics (8) for storing data.

## Revendications

1. Dispositif de mesure (1) configuré pour mesurer l'état d'une matière à mesurer
composée d'huiles ou de graisses destinées à la transformation de denrées alimentaires, avec une tête de mesure (12) pour mesurer une propriété électrique de la matière à mesurer et avec un boîtier (10), **caractérisé en ce que** la tête de mesure (12) est disposée à une extrémité libre d'un épaulement (11) du boîtier (10), qu'une unité d'analyse et de commande électronique (8) est disposée dans le boîtier et que l'épaulement (11) se présente sous une forme ayant une longueur de 15 cm à 40 cm pour empêcher les influences thermiques, sur l'unité d'analyse et de commande électronique (8), lesquelles agissent sur la tête de mesure (12) en raison de la mesure, sachant qu'une unité de saisie (3) pour la saisie de données et une unité de sortie (2) pour la sortie du résultats de mesure sont attribuées à l'unité d'analyse et de commande électronique (8), et sachant qu'un afficheur est en outre attribué à l'unité de sortie (2) pour afficher des paramètres saisis à l'unité d'entrée (3) et/ou pour afficher les résultats de mesure.

2. Dispositif de mesure selon la revendication 1, **caractérisé en ce que** l'épaulement (11) au boîtier (10) est un élément en forme de barre ou de tube, qui est en relation avec le boîtier (10) à l'un de ses côtés et qui, à son autre côté, porte la tête de mesure (12) du dispositif de mesure et se présente sous une forme à résistance thermique.

3. Dispositif de mesure selon l'une quelconque des revendications 1 ou 2, **caractérisé en ce que** l'épaulement (11) se présente sous une forme en tube et accueille en son intérieur des moyens de connexion (50, 60) pour connecter la tête de mesure (12) avec l'unité d'analyse et de commande (8).

4. Dispositif de mesure selon l'une quelconque des revendications 1 à 3, **caractérisé en ce que** l'épaulement (11) présente une longueur comprise entre 25 cm et 35 cm.

5. Dispositif de mesure selon l'une quelconque des revendications 1 à 4, **caractérisé en ce que** l'épaulement (11) présente une propriété de matériau telle que la température à la tête de mesure (12) n'ait aucun effet sur l'unité d'analyse et de commande électronique (8).

6. Dispositif de mesure selon l'une quelconque des revendications 1 à 5, **caractérisé en ce que** la tête de mesure (12) porte un capteur (5) pour mesure les propriétés électriques de la matière à mesurer ainsi qu'un compensateur (6), qui est capable de compenser l'effet de la température sur le capteur (5) en enregistrant lesdits effets et en transmettant ces derniers à l'unité d'analyse et de commande électronique (8), sachant que ledit compensateur (6) subit un changement en cas de modification de sa température et que ledit changement est enregistré par l'unité d'analyse et de commande électronique (8).

7. Dispositif de mesure selon la revendication 6, **caractérisé en ce que** le capteur (5) se présente sous une forme lui permettant de mesurer les constantes diélectriques de la matière à mesurer.

8. Dispositif de mesure selon la revendication 7, **caractérisé en ce que** le capteur (5), pour la mesure des constantes diélectriques, comporte deux électrodes, dont l'une au moins se compose d'un fil métallique mince disposé d'une manière isolée sur une plaque de support (4).

9. Dispositif de mesure selon la revendication 8, **caractérisé en ce que** le fil métallique est un fil en or.

10. Dispositif de mesure selon l'une quelconque des revendications 8 ou 9, **caractérisé en ce que** la plaque de support (4) se compose de céramique.

11. Dispositif de mesure selon l'une quelconque des revendications 1 à 10, **caractérisé en ce qu'**au moins l'épaulement (11), la tête de mesure (12), la plaque de support (4) ou le capteur (5) se compose d'un matériau agréé pour l'utilisation en relation avec des denrées alimentaires.

12. Dispositif de mesure selon la revendication 11, **caractérisé en ce que** le matériau est résistant à la chaleur jusqu'à 230 °C.

13. Dispositif de mesure selon l'une quelconque des revendications 6 à 10, **caractérisé en ce que** la tête de mesure (12) comporte un revêtement (13) pour le capteur (5), lequel revêtement (13) protège le capteur (5) contre le contact avec la paroi ou le fond d'un récipient contenant la matière à mesurer.

14. Dispositif de mesure selon la revendication 13, **caractérisé en ce que** le revêtement (13) se présente sous forme d'arête surplombant le capteur (5).

15. Dispositif de mesure selon l'une quelconque des revendications 1 à 14, **caractérisé en ce que** l'unité de sortie (2) se présente sous une forme commutable pour afficher les résultats de mesure, au choix, sous la forme d'une valeur numérique ou sous forme graphique, par exemple sous la forme d'un graphe en barre.

16. Dispositif de mesure selon l'une quelconque des revendications 1 à 15, **caractérisé en ce que** des moyens de mémorisation (83) sont attribués à l'unité d'analyse et de commande électronique (8) pour la mémorisation, par exemple, de données d'étalonnage ou de correction.

17. Dispositif de mesure selon l'une quelconque des revendications 1 à 16, **caractérisé en ce que** l'unité d'analyse et de commande électronique (8) comporte un microcontrôleur (81).

18. Dispositif de mesure selon la revendication 17, **caractérisé par** une interface de test et de programmation (85), à laquelle peut être raccordé un PC, sachant que ladite interface de test et de programmation (85) est à son tour reliée au microcontrôleur (81).

19. Dispositif de mesure selon l'une quelconque des revendications 1 à 18, **caractérisé en ce qu'**un EEPROM (83) est attribué à l'unité d'analyse et de commande électronique (8) pour la mémorisation de données.
